# EUROPEAN PATENT APPLICATION

(11) **EP 3 831 449 A1**
(43) Date of publication of application: **09.06.2021**
(21) Application number: 19383077.5
(22) Date of filing: 04.12.2019
(51) Int. Cl.: A61P 35/00, C07K 7/00, C07K 14/245, C07K 16/12

(54) **TOOLS AND METHODS TO DETECT AND ISOLATE COLIBACTIN PRODUCING BACTERIA**

(71) Applicant: Consejo Superior de Investigaciones Científicas (CSIC), 28006 Madrid (ES); Fundación Científica de la Asociación Española Contra el Cáncer, 28045 Madrid (ES)
(72) Inventor: SÁNCHEZ GARCÍA, Borja, 33300 Villaviciosa (Principado de Asturias) (ES); MARCOS FERNÁNDEZ, Raquel, 33300 Villaviciosa (Principado de Asturias) (ES); BLANCO MÍGUEZ, Aitor, 33300 Villaviciosa (Principado de Asturias) (ES); MARGOLLES BARROS, Abelardo, 33300 Villaviciosa (Principado de Asturias) (ES)
(74) Representative: Pons

(57) **Abstract**

The invention relates to the field of medical diagnosis, particularly to methods and antibodies useful for the identification of colibactin producing bacteria (pks+ bacteria). Herein are disclosed peptides and antibodies that allow for the detection and isolation of pks+ bacteria, as well as uses and methods of use of said peptides and antibodies.

## Description

The invention relates to the field of medical diagnosis, particularly to methods and antibodies useful for the identification and isolation of colibactin producing bacteria.

### BACKGROUND ART

Cyclomodulins are bacterial toxins that interfere with the eukaryotic cell cycle both by inhibiting or promoting cell proliferation. Amongst these bacterial toxins is Colibactin, identified in 2006 from an *Escherichia coli* strain in a neonatal bacterial meningitis sample. The Colibactin is a toxin produced by a group of **p**oly**k**etide **s**ynthases (*pks*), non-ribosomal peptide synthases and hybrid enzymes which are encoded by a genomic island - the *pks* genomic island. The *pks* genomic island has been reported to be present in several species of bacteria (*pks*+ bacteria), namely *Escherichia coli, Klebsiella pneumoniae, Enterobacter aerogenes, Enterococcus faecium* and *Citrobacter koseri.*

The *pks* genomic island hosts 23 genes, and mutation of any one of these genes, with the exception of Colibactin S - ClbS (and the integrase and transposase proteins), may result in a decrease or complete loss of the genotoxic activity. The *pks* genomic island codes for multienzymatic biosynthesis machinery, including nonribosomal peptide synthetases (NRPSs), polyketide synthases (*PKS*s), an efflux pump, and additional enzymes. The NRPSs and *PKS*s function as a multimodular assembly line that synthesizes an inactive precolibactin. Precolibactin biosynthetic intermediates are offloaded from the assembly line by the ClbQ thioesterase, thus possibly regulating colibactin synthesis and genotoxic activity. Once precolibactin synthesis is finished, the prodrug is taken in charge by ClbM, a multidrug and toxic compound extrusion transporter, and released into the periplasmic space. Once into the periplasmic space, precolibactin is matured by the ClbP peptidase, which will generate the mature colibactin via removal of the N-myristoyl-D-Asn side chain. As a supplemental self-protection mechanism, the *pks* island encodes the ClbS resistance protein, a cyclopropane hydrolase that inactivates colibactin in the producing bacteria (Faïs et al., Toxins 2018, 10, 151, 1 - 16).

Amongst *pks*+ bacteria, the *E. coli pks* genomic island has been the most studied. *E. coli* strains can be divided into eight phylogenetic lineages, A, B1, B2, C, D, E and F, being that the majority of strains belong to the phylogroups A, B1, B2 and D. Amongst the phylogroup B2 are the so called "extra-intestinal pathogenic *E. coli",* the most common gram-negative bacterial pathogen in humans, a group which is highly associated with the presence of the *pks* genomic island. *E. coli pks* genomic island harbouring bacteria have been isolated from intestinal microbiota as a commensal bacterium but also from infectious diseases such as septicaemia, new born meningitis and urinary tract infections. Specifically, in new born sepsis a functional *pks* island contributes to the virulence of the strain and to the colonization of the gut and subsequent transfer to the blood. *pks*+ bacteria in human individuals have been associated with defects in eukaryotic cells, namely cell cycle arrest, chromosome instability, DNA double-strand breakages and chromosome aberrations. In addition, *E. coli pks+* strains are associated with colorectal cancer, with biopsies showing a high degree of colonization of colorectal cancer tissue with *E. coli pks+* strains, suggesting an involvement of *pks+ E. coli* in the development of the cancer.

Despite the growing evidence that the presence of a *pks* island is associated with highly virulent bacteria strains, very few tools exist to target, identify and isolate such bacteria. So far, most tools to identify *pks*+ bacteria strains in isolated samples are based on DNA based techniques (e.g., PCR, sequencing), where the presence of specific *pks* genomic island genes, particularly the ClbB gene which is present in all *pks* genomic islands, are used to determine which strains are *pks*+ strains. However, since mutation of individual genes of the *pks* genomic island revealed that all of the genes are essential for activity, it cannot be deduced that the presence of this gene implies that the strain is in reality a *pks+* strain. In addition, despite being sensitive and accurate, such methods based in DNA techniques require technical trained personnel as well as specialized and expensive hardware, are time consuming and do not allow to determine the presence of live *pks*+ bacteria in the sample. WO2019044736A1 discloses a detection probe for detecting colibactin and *pks+* bacteria in faecal samples (particularly dry faecal samples) and colorectal cancer tissue samples by using a myristoyl asparagine compound, a prodrug motif of colibactin. WO2019044736A1 reveals a fluorescent probe, with several variants, that detects colibactin-producing bacteria by detecting the colibactin producing enzyme ClbP, a peptidase which cleaves the amide bond that links the target molecule Colibactin to the myristoyl asparagine compound. While such probe allows for the detection of live bacteria in isolated samples it has less sensitivity than DNA based methods, as in order for the fluorescence signal to be detected a considered level of bacterial presence is required, leading to the increase in false negative outcomes. In addition, the molecular compound does not allow for the isolation of *pks*+ bacterial cells.

US2009324633A1 discloses various genes that can be used in immunogenic compositions specific for extraintestinal pathogenic *E. coli* strains. US2009324633A1 discloses that polypeptide fragments of the amino acid sequence encoded by such genes can be used to create an immune response in patients or to create immunogens and antibodies, particularly of genes that locate to the outer or inner membrane or in the periplasm. US2009324633A1 does not make a specific reference to *E. coli pks+* bacteria and therefore does not mention which genes should be targeted to obtain an immune response or immunogens specific for the *E. coli pks*+ bacteria.

Therefore, alternative tools for the detection of *pks*+ bacteria are required, tools that allow the detection of *pks*+ bacteria with high sensitivity and specificity. In addition, such tools must allow for the detection of *pks*+ bacteria without the requirement for specialized and expensive hardware, as well as allow for the isolation of *pks*+ bacteria from isolated samples.

### DESCRIPTION OF THE INVENTION

To solve the aforementioned problems with the existing tools for detecting *pks*+ bacteria, the inventors disclose herein isolated peptides originating from the extracellular domain of *pks* proteins, which are unique to *pks*+ bacteria and can be used in the identification of *pks*+ bacteria. In addition, disclosed herein are antibodies that target the mentioned peptides and which permit both the identification and the isolation of *pks*+ bacteria, allowing the depletion and enrichment of *pks*+ bacteria from isolated samples. Furthermore, the tools disclosed herein allow for the identification and isolation of *pks*+ bacteria with methods and kits that do not require specialized and expensive hardware or specialized personnel.

Making use of a dataset of 6212 proteome sequences of *E. coli* strains isolated from the human gut microbiota, the inventors searched for strains with a *pks* genomic island obtaining a set of over 1000 strains. From these, the inventors determined the distribution of the *pks* genomic island genes. Armed with these data, together with a list of peptides 20 amino acids long, obtained from the manual digestion of the proteins from the *pks* genomic island of the *E. coli* strain Nissle 1917, the inventors determined which peptides were present and unique to all strains with a *pks* genomic island not only of *E. coli* but of other bacteria which also possess the *pks* genomic island. From these the authors searched for peptide sequences that were accessible from the outside of the bacteria, i.e. present in the extracellular region of (trans)membrane proteins, namely present in protein regions exposed to the periplasm or to the outside of outer cytoplasm membrane. The inventors obtained 4 peptides in these conditions, all of which could be potentially used to identify *pks*+ bacteria. Therefore, the present invention relates to the usefulness of unique isolated peptides to identify specific bacterial strains, namely, *pks*+ bacterial strains.

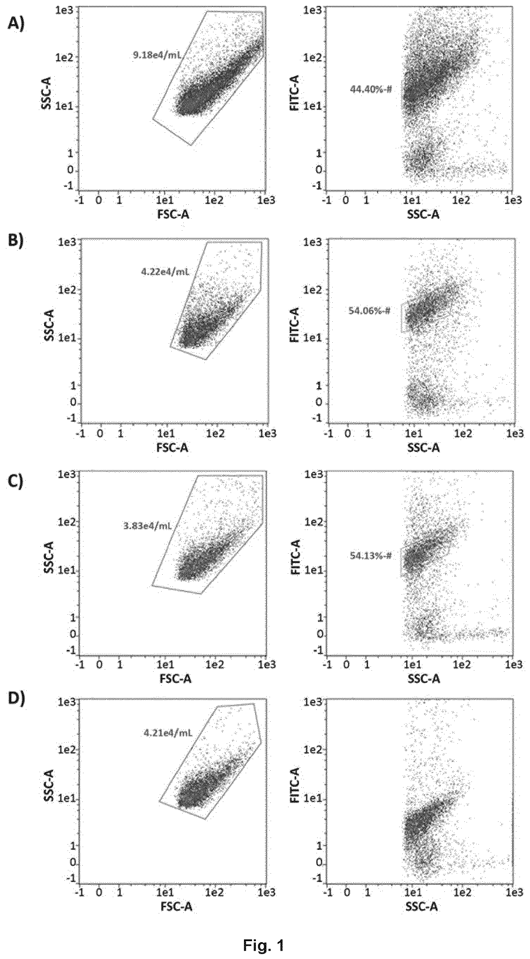

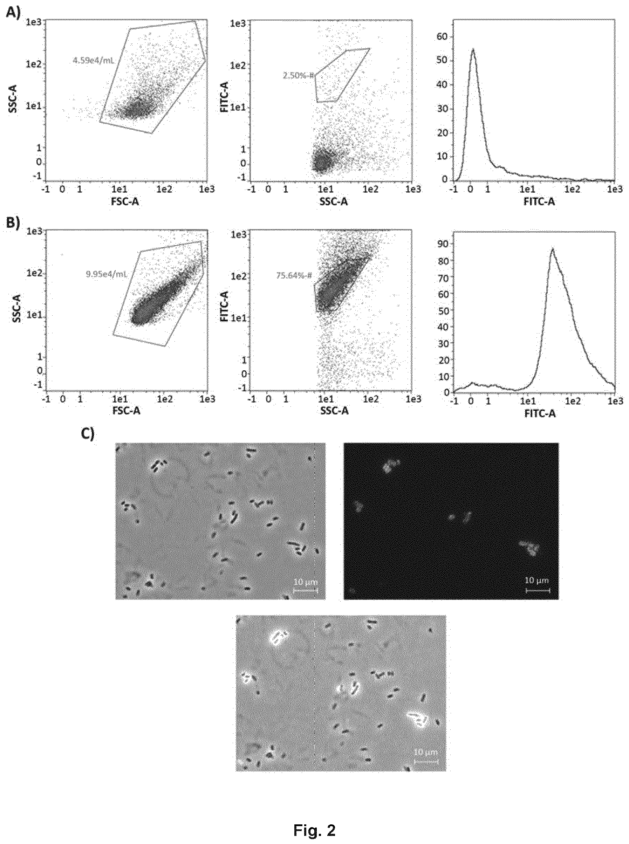

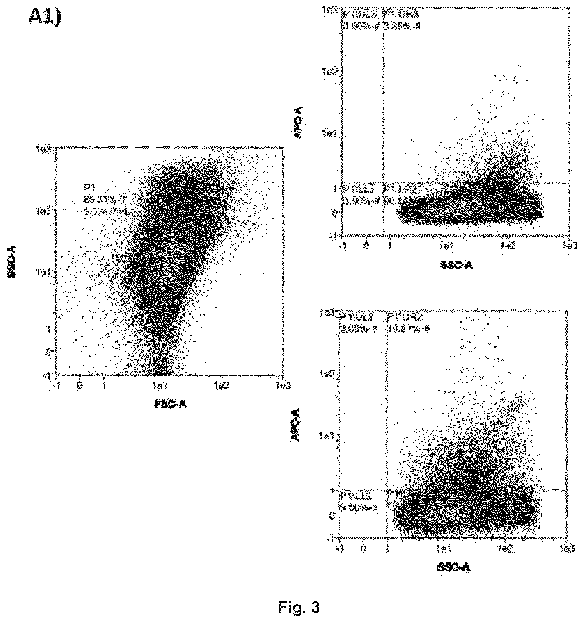

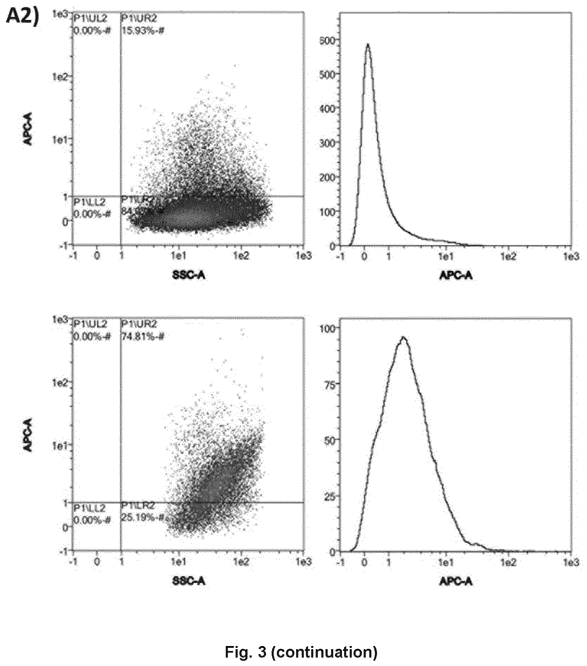

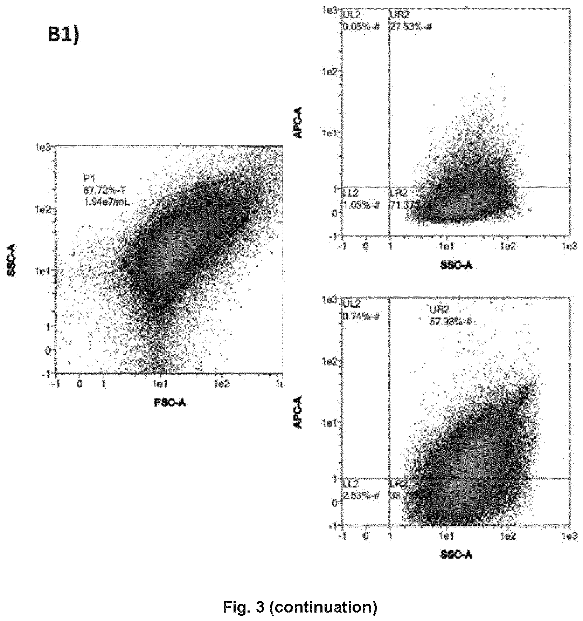

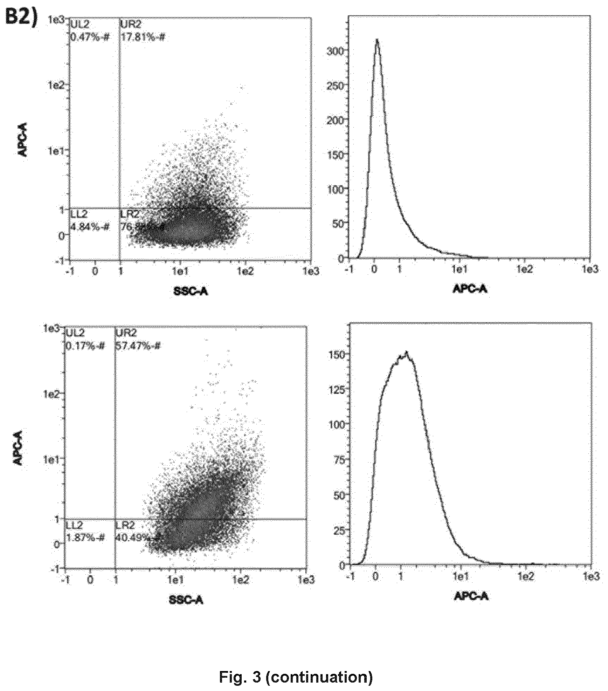

The inventors further created specific antibodies targeting each of the peptides to be able to not only identify but also isolate, deplete and/or enrich *pks*+ bacterial strains from isolated samples. Flow cytometry sorting (FCS) experiments by the inventors showed that from the 4 antibodies created, only 3 had high specificity for *E. coli pks+* strains (Fig. 1). The inventors further labelled the antibodies with different molecules that allowed for detection of *pks*+ bacteria by FCS and fluorescence imaging and for isolation of *pks*+ strains from other microbiota present in the isolated samples tested. The inventors tested the three remaining antibodies in different mediums and at different optical densities of cultures to determine the best conditions for detection of *pks*+ bacteria for all antibodies. Surprisingly one of the antibodies had a better performance compared to the others, including compared to an antibody targeting the same extracellular region of the ClbH protein. Therefore, the inventors decided to use said antibody for the remaining experiments. The inventors showed that the antibody successfully detected *E. coli pks*+ strains, both with FCS and fluorescence imaging (Fig. 2), as well as allowed the isolation of *pks*+ strains from gut microbiota samples (Fig. 3) using magnetic tools to perform the isolation of the bacteria. Therefore, the present invention relates to an antibody targeting a unique peptide for *pks*+ bacteria as a tool for the identification of *pks*+ bacterial strains in isolated samples. Additionally, the present invention relates to the usefulness of antibodies labelled with specific molecules which permit the manipulation of *pks*+ bacterial strains in isolated samples.

Therefore, an aspect of the present invention relates to an isolated peptide consisting of the amino acid sequence SEQ ID NO: 2, hereinafter the "peptide of the invention".

The term "peptide" as used herein refers to a series of amino acid residues, connected one to the other typically by peptide bonds between the alpha-amino and carbonyl groups of the adjacent amino acids. The length of the peptide is not critical to the invention as long as the correct epitopes are maintained. The peptides are preferably 20 amino acids in length, but can be as short as 15 amino acids in length, and as long as 16, 17, 18, 19, 20, 21, 22, 23, 24 or 25. Furthermore, the term "peptide" shall include salts of a series of amino acid residues, connected one to the other typically by peptide bonds between the alpha-amino and carbonyl groups of the adjacent amino acids. Preferably, the salts are pharmaceutical acceptable salts of the peptides, such as, for example, the chloride or acetate (trifluoroacetate) salts. It has to be noted that the salts of the peptides according to the present invention differ substantially from the peptides in their state(s) *in vivo,* as the peptides are not salts *in vivo.*

The term "amino acid" as used herein refers to naturally occurring and synthetic amino acids, as well as amino acid analogues and amino acid mimetics that function similarly to the naturally occurring amino acids. Amino acid may be either L-amino acids or D-amino acids. Naturally occurring amino acids are those encoded by the genetic code, as well as those modified after translation in cells (e.g., hydroxyproline, gamma-carboxyglutamate, and O-phosphoserine). The term "amino acid analogue" as used herein refers to compounds that have the same basic chemical structure (an alpha carbon bound to a hydrogen, a carboxy group, an amino group, and an R group) as a naturally occurring amino acid but have a modified R group or modified backbones (e.g., homoserine, norleucine, methionine, sulfoxide, methionine methyl sulfonium). The term "amino acid mimetic" as used herein refers to chemical compounds that have different structures but similar functions to general amino acids.

The term "isolated" as used herein refers to that the material is removed from its original environment (e.g., the natural environment, if it is naturally occurring). For example, a naturally-occurring peptide or polynucleotide present in a living animal is not isolated, but the same peptide or polynucleotide, separated from some or all of the coexisting materials in the natural system, is an "isolated peptide" or "isolated polynucleotide".

The peptide of the invention (SEQ ID NO: 2) corresponds to the amino acid sequence of the colibactin non-ribosomal peptide synthetase ClbH protein of *E. coli* (SEQ ID NO: 5 and as set forth with the accession numbers WP_063121474 from NCBI Protein database and Q0P7J8 from Uniprot) from positions 402 to 421, which locates in the extracellular C-terminus region of the protein.

Another aspect of the present invention refers to isolated peptides consisting of SEQ ID NO: 1 or SEQ ID NO: 3. The peptide according to SEQ ID NO: 1 corresponds to the amino acid sequence of the ClbH protein of *E. coli* (SEQ ID NO: 5 and as set forth with the accession numbers WP_063121474 from NCBI Protein Database and Q0P7J8 from Uniprot) from positions 342 to 361, which locates in the extracellular C-terminus region of the protein. The peptide according to SEQ ID NO: 3 corresponds to amino acid sequence of the colibactin polyketide synthase ClbC protein of *E. coli* (SEQ ID NO: 6 and as set forth with the accession numbers WP_059348200 from NCBI Protein Database and Q0P7J3 from Uniprot) from positions 261 to 280, which locates in the extracellular N-terminus region of the protein. Therefore, the scope of the present invention also includes the ClbH and ClbC protein amino acid sequences as set forth in SEQ ID NO: 5 and SEQ ID NO: 6 as well as the nucleotide sequences that codify for the respective amino acid sequences.

Another aspect of the present invention relates to an isolated polynucleotide, hereinafter the "polynucleotide of the invention", encoding for the peptide of the invention.

The term "polynucleotide", "nucleotide sequence", "sequence of nucleotides", "nucleic acid" or "oligonucleotide" as used herein refers to a polymeric form of nucleotides of any length, either deoxyribonucleotides or ribonucleotides or analogues thereof, that may or may not be chemically or biochemically modified. Polynucleotides can have any three-dimensional structure and may perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogues. If present, modifications to the nucleotide structure can be imparted before or after assembly of the polynucleotide. The sequence of nucleotides can be interrupted by non-nucleotide components. A polynucleotide can be further modified after polymerization, such as by conjugation with a labelling component. The term also refers to both double- and single-stranded molecules. Unless otherwise specified or required, any embodiment of this invention that is a polynucleotide encompasses both the double-stranded form and each of two complementary single-stranded forms known or predicted to make up the double-stranded form.

The polynucleotide of the present invention may comprise other elements apart from the encoding sequence, such as, for example, but not limited to, introns, noncoding sequences at terminal ends 5' or 3', ribosome binding sites or stabilizing sequences. These polynucleotides may also include encoding sequences for additional amino acids that may be useful, for example, for increasing the stability of the peptide generated therefrom or enable simplified purification thereof.

A polynucleotide is composed of a specific sequence of four nucleotide bases: adenine (A); cytosine (C); guanine (G); thymine (T); and uracil (U) for thymine when the polynucleotide is RNA. Thus, the term "polynucleotide sequence" is the alphabetical representation of a polynucleotide molecule. Such polynucleotides could be part of a vector and/or such polynucleotides could be part of a composition, and still be isolated in that such vector or composition is not part of its natural environment.

A further aspect of the invention relates to isolated polynucleotides which encode for the peptides consisting of SEQ ID NO:1 or SEQ ID NO:3.

Another aspect of the invention refers to a gene construct, preferably a cloning or expression vector, that comprises any of the polynucleotides disclosed herein or any combination thereof. The expression vector enables the replication and expression of the polynucleotide of the invention in the interior of a cell.

The term "expression vector" or "cloning vector" relates to a nucleic acid construct, preferably a DNA construct, wherein the polynucleotide of the invention can be integrated without losing its replication capacity. The expression vector is designed to direct the transformation or transduction of said polynucleotide in the interior of a host cell and its subsequent replication and expression. In addition to the polynucleotide, the vector comprises other genetic elements sequentially oriented (operably linked) in such a manner that the polynucleotide may be transcribed and translated in the cell. Said genetic elements may be, but not limited to, a promoter, a terminator, a leader sequence, an enhancer, a transcription initiation site, one or more replication origins, an untranslated regulatory region 3' and/or 5', a potentiator, a polyadenylation signal or any other control sequence. The expression vector may also comprise sequences that direct the expression of the polynucleotide comprised therein in a specific cell type, in a specific cell sublocation or in a specific tissue.

The expression vector can be selected, but not limited to, from a plasmid, phage, cosmid, phagemid, autonomously replicating sequence (ARS), yeast artificial chromosome (YAC), bacterial artificial chromosome (BAC), human artificial chromosome (HAC) or viral vectors such as, but not limited to, lentivirus, baculovirus, adenovirus, retrovirus, adeno-associated viral vector (AAV) or any other type of DNA molecule capable of replicating in the interior of a prokaryotic or eukaryotic cell, preferably eukaryotic cell.

The expression vector may be a self-replicating vector, whose replication is independent from the genome of the cell in which it is introduced, or may be a vector which becomes integrated in the genome of the host cell once transfected and replicates therewith. Thus, the vector may comprise sequences that facilitate its insertion in a specific location within the genome of the host cell.

A further aspect of the present invention relates to an antibody that specifically recognizes the peptide consisting of SEQ ID NO: 1, SEQ ID NO: 2 or SEQ ID NO: 3, preferably the peptide of the invention consisting of SEQ ID NO: 2. In a preferred embodiment, the antibody is conjugated to a detectable label.

The term "antibody" relates to immunoglobulin molecules or to immunologically active portions of immunoglobulin molecules, i.e. molecules containing an antigen fixation site which binds specifically (immune reacts) to any one of the peptides described herein, preferably to the peptide of the invention consisting of SEQ ID NO: 2. Examples of portions of immunologically active immunoglobulin molecules include fragments F(ab) and F(ab')2, which may be generated by treating the antibody with an enzyme such as pepsin or recombinantly.

The antibodies mentioned in the present invention are, preferably, of IgG isotype.

The antibodies of the invention may be polyclonal (typically includes different antibodies aimed at different determinants or epitopes) or monoclonal (aimed at a single determinant in the antigen). The expression "monoclonal antibody" alludes to a population of antibody molecules containing only one species of an antigenic fixation site capable of immune reacting with a particular epitope of the antigen. The monoclonal antibody may be biologically altered by means of genetic manipulation, or may be synthetic, possible lacking the antibody, in its entirety or in parts, portions that are not necessary for recognising the peptide of the invention, or any of the peptides consisting of SEQ ID NO: 1 or SEQ ID NO: 3, and being substituted by others that transmit additional advantageous properties to the antibody. In a preferred embodiment, the antibody of the invention is a polyclonal antibody.

The antibodies of the invention may also be recombinant, humanized, chimeric, synthetic or a combination of any of the foregoing. A "recombinant antibody" (rAC) is an antibody which has been produced in a host cell which has been transformed or transfected by a polynucleotide that encodes any one of the peptides consisting of SEQ ID NO: 1 to 3, preferably the peptide of the invention consisting of SEQ ID NO: 2; by a nucleic acid encoding any of the antibodies that specifically recognize one of the peptides consisting of SEQ ID NO: 1 to 3, preferably the antibody of the invention that specifically recognizes the peptide consisting of SEQ ID NO: 2; or any one of the peptides consisting of SEQ ID NO: 1 to 3, preferably the peptide of the invention consisting of SEQ ID NO: 2, as a result of a homologous recombination. Said host cell includes a cell in a cellular culture *in vitro* and a cell in a host animal.

The antibodies of the invention may also be chimeric. Thus, a region of the heavy and/or light chain is identical or homologous to the corresponding sequences in antibodies from a certain species or belonging to a class or subclass of certain antibodies, while the remaining chain(s) are identical or homologous to the corresponding sequences in antibodies derived from other species or belonging to another class or subclass of antibodies, and to fragments of said antibodies, in such a manner as to exhibit the desired biological activity.

In a preferred embodiment, the antibody that specifically recognizes the peptide consisting of SEQ ID NO: 2 comprises a variable region comprising, more preferably consisting of, the SEQ ID NO: 7 (WRDGESQQIHYIGRNDFQIK).

The expressions antibody "against the", "that targets", "that reacts to", "specific for", "that specifically recognizes" the peptide, can be used interchangeably. The specificity of the antibody for its antigen is the capacity of the same to bind the peptide disclosed in this invention with a binding affinity that is preferably at least 2-fold, 50, 100 or 1,000-fold higher than its binding affinity for a non-specific antigen different from the peptide disclosed herein.

The term "detectable label" as used herein refers to molecules that can generate a signal which is detectable by a method suitable for such detection. Methods for detection of labels include, but are not limited to, fluorescence, light, confocal and electron microscopy; magnetic resonance imaging and spectroscopy; fluoroscopy, computed tomography and positron emission tomography, enzyme-linked immunosorbent assays, fluorescence-detection size-exclusion chromatography, lateral flow assay, radioimmunoassay, radiolabelling, western blotting, immunohistochemistry, immunofluorescence, immunocytochemistry, flow cytometry, fluorescence activated cell sorting, immunoprecipitation and enzyme liked immunospot. Suitable labels include, but are not limited to, fluorescein, rhodamine, eosin and other fluorophores, radioisotopes, gold, gadolinium and other lanthanides, paramagnetic iron, fluorine-18 and other positron-emitting radionuclides. Additionally, probes or labels may be bi- or multi-functional and be detectable by more than one of the methods listed. In a preferred embodiment of the present invention, the detectable label is a fluorescence dye suitable to be detected by flow cytometry. In a more preferred embodiment of the present invention, the detectable label is selected from the list consisting of: fluorescein isothiocyanate (FITC), allophycocyanin (APC), DyLight 405, Alexa Fluor 405, Alexa Fluor 488, DyLight 550, Pacific Blue, phycoerythrin, Alexa Fluor 647, DyLight 650, peridinin chlorophyll protein, Alexa Fluor 700, EGFP, CFP, YFP, RFP, mCherry, biotin, horseradish peroxidase (HRP), gold beads and paramagnetic iron.

The term "conjugated" refers to any method known in the art for functionally connecting protein domains with labels, including without limitation recombinant fusion with or without intervening domains, intein-mediated fusion, non-covalent association, and covalent bonding, e.g., disulfide bonding peptide bonding, hydrogen bonding, electrostatic bonding, and conformational bonding, e.g., biotin-avidin associations. The conjugation to a label can be either by chemical or recombinant means. Chemical means refers to a reaction between the antibody and the label such that there is a covalent bond formed between the two molecules to form one molecule.

In another preferred embodiment, the antibody of the invention is immobilized in a support, for instance, a matrix surface (preferably a nylon matrix), a membrane; a glass, silicon, graphene or plastic support; a plate, preferably a microtiter plate; nanotechnology-based supports, particles, preferably nanoparticles or magnetic particles; carbon derivatives and others, or on beads for example agarose spheres or superparamagnetic microspheres formed by biodegradable matrixes. The term "immobilized" means that the antibody is linked to any support or surface without losing its activity or antigen binding properties.

A further aspect of the present invention is a pharmaceutical composition, hereinafter the "pharmaceutical composition of the invention", comprising the antibody of the present invention, preferably the antibody that specifically recognizes the peptide consisting of SEQ ID NO: 2, and preferably a pharmaceutical acceptable adjuvant, a pharmaceutical acceptable vehicle and/or another active principle.

The term "pharmaceutical acceptable adjuvant" or "excipients" as used herein makes reference to a substance that aids the absorption of the elements of the composition of the invention, stabilizes said elements, activates or aids the preparation of the composition in the sense of giving it consistency or providing flavours that make it more pleasant. Thus, the excipients could have the function of maintaining the ingredients bound together, such as for example in the case of starches, sugars or celluloses, the function of sweetening, the function of colouring, the function of protecting the composition, such as for example, to insulate it from air and/or humidity, the function of filling a tablet, capsule or any other form of presentation, a disintegratory function for facilitating the dissolution of the components and its absorption in the intestine, without excluding other types of excipients not mentioned in this paragraph.

The term "pharmaceutically acceptable vehicle" is a substance used in the composition to dilute any of the components comprised therein up to a certain volume or weight. The pharmaceutically acceptable vehicle is an inert substance or of identical action to any of the elements comprised in the composition of the present invention. The function of the vehicle is to facilitate the incorporation of other elements, allow better dosing and administration or give the composition consistency and form.

The composition of the present invention may be formulated for administration thereof in a variety of forms known in the state of the art. Examples of preparations include any solid composition (tablets, pills, capsules, granules, etc.) or liquid (solutions, suspensions or emulsions) for oral, topical or parenteral administration. The composition of the present invention may also be in the form of sustained-release formulation of drugs or of any other conventional release system, whether contained, but not limited to, nanoparticles, liposomes or nanospheres, in a polymer material, in a biodegradable or non-biodegradable implant or in biodegradable microparticles, such as for example, biodegradable microspheres. Such composition and/or its formulations may be administered to an animal and, therefore, to humans, in a variety of forms, including, but not limited to, intraperitoneal, intravenous, intradermal, intraspinal, instrastromal, intraarticular, intrasinovial, intrathecal, intralesional, intraarterial, intramuscular, intranasal, intracranial, subcutaneous, intraorbital, intracapsular, topical, by means of transdermal patches, percutaneous, nasal spray, surgical implant, internal surgical painting or infusion pump.

Yet another aspect of the present invention relates to a kit, hereinafter the" kit of the invention", comprising any of the peptides described herein or any combination thereof, preferably the peptide consisting of SEQ ID NO: 2, and/or the antibody of the invention, preferably the antibody that specifically recognizes the peptide consisting of SEQ ID NO: 2.

The kit of the invention comprises at least the peptide of the invention and/or the antibody of the invention. The kit may further include other components that find use in the subject invention, e.g., buffers, delivery vehicles, antibody supports, positive and/or negative controls components optionally wherein such controls comprise antibodies, containers with solutions or empty that encounter use in the practicing of the subject invention. In a preferred embodiment the antibody of the invention is immobilized in a suitable support wherein the support with the immobilized antibody is contained in the kit. In another preferred embodiment the antibody is labelled, e.g. a magnetic label, and the materials required to detect such label are included in the kit, e.g. magnetic beads. In yet another preferred embodiment the kit contains one or more of the antibodies that recognize the peptides consisting of SEQ ID NO: 1 to 3. The various components of the kit may be present in separate containers or certain compatible components may be combined into a single container, as desired. In addition to the above components, the subject kits will further include instructions for practicing the subject invention. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g. a CD, an USB, etc., on which the information has been recorded. Yet another means that may be present is a website address which may be used via the internet to access the information at a removed site. Any convenient means may be present in the kits.

As shown in the examples below, an antibody generated against any one of the peptides consisting of SEQ ID NO: 1 to 3, preferably against the peptide of the invention consisting of SEQ ID NO: 2, is capable of identifying *pks*+ bacterial strains preferably by both fluorescence cell sorting as well as by fluorescence microscopy. In addition, the antibody of the invention also allows, preferably in its labelled form, to perform the isolation of *pks*+ bacterial strains from isolated samples as well as the enrichment/depletion of *pks*+ bacteria in isolated samples. Therefore, a further aspect of the invention relates to the use of the antibody of the invention or the kit of the invention for the *in vitro* detection in isolated samples of *pks*+ bacteria. In a preferred embodiment, the *pks*+ bacteria are selected from the list consisting of: *Escherichia sp., Klebsiella sp., Enterobacter sp., Enterococcus sp., Citrobacter sp.* and *Shigella sp.* In a preferred embodiment the *pks*+ bacteria are *Escherichia coli, Klebsiella pneumoniae, Enterobacter aerogenes, Enterobacter cloacae, Enterococcus faecium, Citrobacter koseri, Citrobacter gillenii and Shigella dysenteriae.* In a more preferred embodiment, the *pks*+ bacteria are *E. coli.*

The term "detection in isolated samples of *pks*+ bacteria" refers to the action or process of identifying the presence of *pks*+ bacterial cells which contain any of the antibodies that target the peptides consisting of SEQ ID NO: 1 to 3, preferably the antibody of the invention that targets the peptide consisting of SEQ ID NO: 2, bound to their surface, using detection methods such as, without limitation, conventional immunoassay such as aggregation reactions where antibody is adsorbed on polystyrene latex particles, ELISA, which is a conventional technology performed in a microtiter plate, conventional immunochromatography methods, sandwich assay, whereby said antibody labelled with coloured particles or particles that have colouring capability, or with enzyme or phosphor, and magnetic microparticles coated with capture antibody. In addition, in the cases where the antibodies are conjugated to a label, the methods to detect said label, as specified previously, are also suitable to perform the detection of the antibody-bacteria conjugates in isolated samples.

Examples of suitable isolated samples include, but are not limited to, bodily tissues, faecal matter and fluids, such as blood, sputum and urine. In a preferred embodiment, the subject-derived cell or tissue sample contains a cell population including an epithelial cell, more preferably a cancerous epithelial cell or an epithelial cell derived from tissue suspected to be cancerous, even more preferably a colon rectal cancerous epithelial cell. Further, if necessary, the cell may be purified from the obtained bodily tissues, faecal matter and fluids, and then used as the subjected-derived sample. In a more preferred embodiment, the isolated sample referred to in the present invention is a faecal sample.

A further aspect of the present invention relates to the use of the antibody of the invention or the kit of the invention for the *in vitro* isolation from isolated samples of *pks*+ bacteria. In a preferred embodiment, the *pks*+ bacteria are selected from the list consisting of: *Escherichia sp., Klebsiella sp., Enterobacter sp., Enterococcus sp., Citrobacter sp.* and *Shigella sp.* In a preferred embodiment the *pks*+ bacteria are *Escherichia coli, Klebsiella pneumoniae, Enterobacter aerogenes, Enterobacter cloacae, Enterococcus faecium, Citrobacter koseri, Citrobacter gillenii and Shigella dysenteriae.* In a more preferred embodiment, the *pks*+ bacteria are *E. coli.*

The term "isolation from isolated samples of *pks*+ bacteria" refers to the action or process of isolating, extracting or selectively accumulate the *pks*+ bacterial cells which contain any of the antibodies that target the peptides consisting of SEQ ID NO: 1 to 3, preferably the antibody of the invention that targets the peptide consisting of SEQ ID NO: 2, more preferably the antibody of the invention labelled, bound to their surface using isolation methods well known in the art such as, without limitation, immunomagnetic separation, fluorescence activated cell sorting, immunodensity cell separation, cell affinity chromatography, electrophoretic sorting, optical sorting, immunoguided laser capture microdissection. In a preferred embodiment, the isolation of *pks*+ bacteria results in the depletion of the *pks*+ bacteria from the isolated sample. In another preferred embodiment, the isolation of the *pks*+ bacteria results in the enrichment of the *pks*+ bacteria in the isolated sample or in another suitable solution to contain the *pks*+ bacteria. Examples of suitable isolated samples are identical to the ones referenced above for detection of *pks*+ bacteria in isolated samples.

The term "depletion" or "enrichment" as used herein refers to a reduction or increase, respectively, of the number of *pks*+ bacterial cells from a cell population, in an isolated sample or in a solution adequate for the presence of bacteria cells. The depletion/enrichment may be based on the number of cells defined by absence/presence of any of the antibodies that recognize one of the peptides consisting of SEQ ID NO: 1 to 3, preferably the antibody of the invention that targets the peptide consisting of SEQ ID NO: 2, more preferably the antibody of the invention labelled.

Yet another aspect of the invention relates to a method, hereinafter the "first method of the invention", for the *in vitro* detection of *pks*+ bacteria, preferably the *pks*+ bacteria selected from the list consisting of: *Escherichia sp., Klebsiella sp., Enterobacter sp., Enterococcus sp., Citrobacter sp.* and *Shigella sp.* In a preferred embodiment the *pks*+ bacteria are *Escherichia coli, Klebsiella pneumoniae, Enterobacter aerogenes, Enterobacter cloacae, Enterococcus faecium, Citrobacter koseri, Citrobacter gillenii and Shigella dysenteriae,* more preferably *E. coli pks*+, comprising:
a. providing an antibody of the invention, preferably the antibody of the invention that targets the peptide consisting of SEQ ID NO: 2;
b. adding the antibody of the invention to a solution containing a sample of interest in sufficient conditions and with sufficient time of incubation to allow the antibody to bind to the target, creating a reaction mixture; and
c. detecting the presence of the bacterial cells bound to the antibody in the reaction mixture.

"Adding the antibody of the invention to a solution containing a sample of interest in sufficient conditions and with sufficient time of incubation to allow the antibody to bind to the target" is understood to be the incubation of the antibody of the invention with a solution which contains an isolated sample of interest which may contain *pks*+ bacteria. The resulting mixture is the reaction mixture. The incubation is understood to be performed with the necessary conditions and sufficient time to allow the formation of antigen-antibody complexes which can subsequently be detected. Furthermore, the reaction mixture is understood to contain any other additives required for the formation of antigen-antibody complexes like, without limitation, buffer and salts.

"Detecting the presence of the bacterial cells bound to the antibody in the reaction mixture" is understood to be the use of a method to detect the presence of antibodies bound to the antigen which is located in the surface of the *pks*+ bacteria. In the case where the antibodies are labelled, the methods described above to detect antibodies and labels bound to antibodies are also applicable, without limitation, to detect bacterial cells with antibodies attached to their surface. Such methods include without limitation, conventional immunoassay such as aggregation reactions where antibody is adsorbed on polystyrene latex particles, ELISA, which is a conventional technology performed in a microtiter plate, conventional immunochromatography methods, sandwich assay, whereby said antibody labelled with coloured particles or particles that have colouring capability, or with enzyme or phosphor, and magnetic microparticles coated with capture antibody, fluorescence, light, confocal and electron microscopy; magnetic resonance imaging and spectroscopy; fluoroscopy, computed tomography and positron emission tomography, enzyme-linked immunosorbent assays, fluorescence-detection size-exclusion chromatography, lateral flow assay, radioimmunoassay, radiolabelling, western blotting, immunohistochemistry, immunofluorescence, immunocytochemistry, flow cytometry, fluorescence activated cell sorting, immunoprecipitation and enzyme-linked immunospot.

Another aspect of the present invention relates to a method, hereinafter the "second method of the invention", for the isolation from isolated samples of *pks*+ bacteria comprising steps a. and b. of the first method of the invention and a step c. comprising separating the cells bound to antibodies from the reaction mixture.

"Separating the cells bound to antibodies from the reaction mixture" is understood to mean the application of a method wherein the *pks*+ bacteria cells which contain the antigen at their surface to which antibodies are bound to during the reaction incubation time, are made to be apart from the remaining of the constituents of the solution. Examples of such methods are, without limitation, fluorescence activated cell sorting, magnetic beads separation, affinity columns. Furthermore, "separating the cells bound to antibodies from the reaction mixture" also refers to the possibility of enrich or deplete the reaction mixture of *pks*+ bacteria cells which contain the antigen at their surface to which the antibodies are bound to during the reaction mixture. Therefore, in a preferred embodiment of the second method, separating the cells bound to antibodies from the reaction mixture results in the depletion of said cells from said reaction mixture. In another preferred embodiment of the second method, separating the cells bound to antibodies from the reaction mixture results in the enrichment of the said cells in the isolated sample or a subsequent adequate solution.

A yet another aspect of the present invention relates to the use of the peptides of the invention in the production of an antibody, preferably an antibody useful for the detection, isolation and/or enrichment/depletion of *pks*+ bacteria in an isolated sample. The peptides of the invention have antigenic capacity, due to which it can be used to immunize an individual, preferably a mammal. Once immunization has taken place, serum is extracted from the immunized animal. The extraction of the serum may be performed, for example, but not limited to, by means of blood extraction, subsequent coagulation thereof, removal of the fibrin clot and other components and isolation of the serum. In order to obtain the serum, anticoagulant is not applied to the blood, but rather is left to coagulate and is centrifuged. "Serum" or "blood serum" is understood to be the blood component resulting from allowing the coagulation thereof and removing the fibrin clot and other components, which contains a number of biological effectors, such as the platelet-derived growth factor, secreted by the cellular components during said coagulation. It is yellow in colour, brighter than plasma. Subsequently, the antibodies present in the serum obtained, which are specific against one of the peptides of the invention, particularly the peptide used in the immunization, are purified. Various antibody purification methods that could be carried out for such purpose are known in the state of the art, such as for example, but not limited to, electrophoretic or chromatographic methods.

The peptide can also be used to immunize a bird. Preferably, after being immunized the eggs of the immunized animal are collected, the yolks are separated, the lipids removed and the antibodies precipitated/purified using various methods known in the state of the art.

"Electrophoresis" is an analytical separation technique based on the movement or migration of macromolecules dissolved in a medium (electrophoresis buffer), by means of a matrix or solid support as a result of the action of an electrical field. The behaviour of the molecule depends on its electrophoretic mobility and this mobility depends on charge, size and shape. There are numerous variations of this technique based on the equipment used, supports and conditions for carrying out the separation of the molecules. Electrophoresis is selected from the list consisting of, but not limited to, capillary electrophoresis, paper electrophoresis, agarose or polyacrylamide gel electrophoresis, isoelectric focusing or bidimensional electrophoresis.

"Chromatographic methods" allow the separation of the molecules according, but not limited to, their charge, size, molecular mass, polarity or redox potential. The chromatographic technique is selected from among, but not limited to, partition chromatography, filtration, adsorption chromatography, molecular exclusion chromatography, ionic exchange chromatography, hydrophobic chromatography or affinity chromatography, and can be executed on columns, paper or plates.

Another aspect of the present invention relates to the antibody of the invention, preferably the antibody of the invention that targets the peptide consisting of SEQ ID NO: 2, or the pharmaceutical composition of the invention for use as a medicament. A further aspect of the present invention relates to the antibody of the invention, preferably the antibody of the invention that targets the peptide consisting of SEQ ID NO: 2, or the pharmaceutical composition of the invention for use in the treatment or prevention of conditions or diseases associated with high colonization by *pks+* bacteria, such as colorectal cancer, septicaemia, new born meningitis and urinary tract infections, preferably colorectal cancer.

The term "medicament", as used in the present description, relates to any substance or combination of substances having properties for the treatment or prevention of diseases in organisms, preferably human beings, or that may be used or administered to the organisms, preferably human beings, with the aim of restoring, correcting or modifying the physiological functions by exercising a pharmacological, immunological or metabolic action. The medicament of the invention may be used either alone or in combination with other medicaments or compositions by way of combined therapy, and can be administered simultaneously or at different time intervals.

The term "treatment", as used in the present invention, relates to combating the effects caused as a consequence of the disease or pathological condition of interest in a subject (preferably a mammal and, more preferably, a human) including:
(i) inhibit the disease or pathological condition, i.e. stop the development thereof;
(ii) alleviate the disease or pathological condition, i.e. cause the regression of the disease or pathological condition or its symptomatology;
(iii) stabilize the disease or pathological condition.

The term "prevention", as used in the present invention, consists of avoiding the onset of the disease, i.e. avoiding the disease or pathological condition in a subject (preferably a mammal and, more preferably, a human), in particular, when said subject has a predisposition to the pathological condition but has not been diagnosed yet.

The term "colorectal cancer" as used herein refers to the well-accepted medical definition that defines colorectal cancer as a medical condition characterized by cancer of cells of the intestinal tract below the small intestine (e.g., the large intestine (colon), including the cecum, ascending colon, transverse colon, descending colon, and sigmoid colon, and rectum). Additionally, as used herein, the term "colorectal cancer" is meant to further include medical conditions which are characterized by cancer of cells of the duodenum and small intestine (jejunum and ileum).

The term "associated with high colonization by *pks*+ bacteria" as used herein refers to the higher prevalence of *pks*+ bacteria in isolated samples, preferably of colorectal cancer material (e.g. colonic lavage samples and/or biopsy samples), from patients compared to isolated samples from non-patients, wherein the patient samples have higher percentage of *pks*+ bacteria compared with the samples of non-patients.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

**Fig. 1****. Dispersion diagrams of representative flow cytometry experiments.** Diagrams show the acquisition of marked *Escherichia coli* Nissle 1917 cell suspension in exponential phase of growth and aeration condition with four different polyclonal antibodies: (A) anti-peptide 1, (B) anti-peptide 2, (C) anti-peptide 3 and (D) anti-peptide 4.
**Fig. 2****. Labelling results obtained in nutrient broth at OD₆₀₀ of 1.00 with antibody anti-peptide 2.** Dispersion diagrams and histograms show the flow cytometry acquisition of *E. coli* (A) LMG2092 and (B) Nissle 1917. (C) Immunofluorescence microscopy photography shows the binding of FITC-anti-peptide 2 antibody to the Nissle strain.
**Fig. 3****. Detection, enrichment and depletion of *E. coli pks*+ from gut microbiotas.** Dispersion diagrams and histograms shows the flow cytometry acquisition in a microbiota from a healthy donor (A) and a patient with Lynch syndrome (B). For each sample, the microbiota without labelling (1-top) and the labelled microbiota (1-bottom) and the negative (2-top) and positive (2-bottom) are shown.

### Examples

### 1. Materials and Methods

### Distribution and structure of the colibactin genome island

Six thousand two hundred twelve *E. coli* strains isolated from humans and deposited in the PATRIC database (https://www.patricbrc.org/) and the 35 reference strains from NCBI were selected for the study of the distribution and structure of the colibactin genome island (*pks*+ strains). For colibactin detection, proteomes from the different strains were aligned against the published sequence of the colibactin genome island of the *E. coli* strain IHE3034 (as set forth with the accession no. AM229678) using BLASTp. For positive detection, 20 out of 23 genes must be detected with less than 5 gaps between consecutive genes. For each gene a cut-off value was set based on the alignment expected value (E-value < 1e⁻⁵) and sequence coverage (identity > 80%).

### Selection of pks+ unique peptides

The proteins of the colibactin genome island of the *E. coli* strain IHE3034 were manually digested in peptides of 20 amino acids. Later, the proteomes of the 6,212 *E. coli* strains from the PATRIC database were aligned against these peptides using BLASTp. For positive detection, a cut-off value was set based on the alignment expected value (E-value < 1e⁻⁵) and sequence coverage (identity > 80%). The 4 peptides, the peptides of the invention, from cytoplasmic membrane proteins with the biggest detection score on the *pks*+ strains and the lowest detection score on the *pks*- strains were selected. Subcellular location of the reference colibactin genome island was predicted using the PSORTb v3.0 tool (https://www.psort.org). Transmembrane prediction was performed using the HMMTOP v2.0 tool (www.enzim.hu).

### Bacterial strains and growth conditions

*Escherichia coli* LMG2092 and Nissle 1917 were grown in Luria-Bertani broth (LB) containing 10 g/L tryptone (Biokar Diagnostics, France), 5 g/L yeast extract (Biokar Diagnostics, France), 10 g/L of NaCl (Merck, KGaA, Darmstadt, Germany) and 1 litre of deionised H₂O. All cultures were grown on the surface of agar plates at 37° C in a MG500 anaerobic chamber (Don Whitley Scientific, West Yorkshire 100, UK) with an atmosphere of 10% (v/v) H2, 10% CO₂, and 80% N2 for 48h. After that, one single colony was inoculated in 4 mL of fresh liquid media and all cultures were incubated at 37°C for 24 h in anaerobic, aerobic and aerobic with shaking conditions. The next day, 100 µL of the bacteria suspension were inoculated into 4 mL of fresh medium and incubated at 37°C until an optical density (OD600) of about 0.6 after 3 h approximately. After that, cultures were harvested by centrifugation, washed once with bacterial flow cytometry buffer (Miltenyi, Bergisch Gladbach, Germany) and resuspended in the same buffer to an OD₆₀₀ = 0.2, which represents around 108 CFUs/mL. The bacteria were also growth in fresh liquid media of Nutrient broth (Oxoid, Ltd., Baingstoke, Hampshire, UK) and nutrient broth supplemented with 2% of glucose (Sigma, St. Loui, MO).

### Faecal sample collection and microbiota separation

The study sample comprised 5 faecal samples from healthy donors and 5 faecal samples from patients with Lynch syndrome. Fresh faecal material from healthy donors was collected in a sterile container and immediately manipulated and homogenised within a maximum of 2 h from defecation. Nine millilitres of sterile NaCl 0.9% (w/v) was added to 1g of sample, and the mixture was homogenised in a sterile bag using a laboratory paddle blender (Stomacher Lab Blender 400, Seward Ltd. UK). Microbiota extraction was then performed following the protocol described by Hevia et al. (Sci Rep. 2015; 5:16807. doi:10.1038/srep16807). A solution of Nycodenz® 80% (w/v) (PROGEN Biotechnik GmbH, Heidelberg, Denmark) was prepared in ultrapure water, and sterilised at 121 °C for 15 min. A volume of 3 mL of the diluted, homogenised faecal sample was placed on top of 1 mL of the Nycodenz® solution, and centrifuged for 40 min at 4 °C (9,000 g, TST41.14 rotor, Kontron, Milan, Italy). The upper phase (soluble debris) was discarded after centrifugation, and the layer corresponding to the microbiota was collected, washed once and resuspended in 1mL of flow cytometry (FC) buffer (1x MACSQuant Running Buffer, Miltenyi Biotec, Germany).

### Polyclonal antibody generation

Polyclonal sera against the purified peptides of the invention from *E. coli* Nissle 1917 were generated in the Central Facilities of the University of Oviedo (Spain). A rabbit was immunised five times, with an interval of 15 days between immunisations, with 500 µg of peptide dissolved in 1 mL of PBS and mixed with 1 mL of Freund's Incomplete Adjuvant. The rabbit was finally sacrificed by intracardiac puncture and blood was let to coagulate at 37 °C for 4 h and subsequent overnight incubation at 4 °C. Serum was separated by centrifugation (30 min, 2,000 g), and used for purifying the IgG. Firstly, ammonium sulphate was added to a final concentration of 45 % (w/v), and the mix was incubated overnight at 4 °C. After centrifugation (1 h, 10,000 g, 4 °C), the pellet was resuspended in 30 mL of PBS. This was extensively dialysed against PBS, and loaded in a ProteinA Sepharose 4 Fast Flow, previously equilibrated with 10 column volumes of PBS (50 mL). The column was washed with 6 column volumes of PBS, and five fractions of 5 mL were eluted with citric acid 100 mM pH 3.0. pH was corrected in each aliquot by adding 1 mL of 1M Tris-HCl pH 9.0. Fractions were mixed, centrifuged in a Vivaspin 20 device (3,000 x g, molecular weight cut-off of 10 kDa) and washed with 20 mL of PBS. Protein concentration was estimated by measuring the A280 and samples were aliquoted and stored at -80 °C.

### Antibody conjugation

Polyclonal antibodies, anti-peptide according to SEQ ID NO: 1, anti-peptide according to SEQ ID NO: 2, anti-peptide according to SEQ ID NO: 3 and anti-peptide according to SEQ ID NO: 4, serum IgG fractions were conjugated with fluorescein isothiocyanate (FITC) and allophycocyanin (APC) using the commercial kits (Abcam Cambridge, MA, USA) and following manufacturer's instructions. For FITC and APC conjugation, the anti-peptide polyclonal antibodies were reconstituted in amine-free phosphate buffered saline (PBS). For each antibody, one hundred millilitres of 1.5 mg/mL antibody were added to the reactive dye for each conjugation. Before adding the antibody to the FITC/APC mix, 10 µL of FITC-Modifier or APC- Modifier reagent was added to the antibody. The antibody-dye mixtures were incubated in the dark at room temperature (20-25°C) for 3 h. After incubation, 10 µL of FITC-Quencher or APC-Quencher reagent was added and mixed gently. The concentration of antibody in the final sample was 20 µg/mL.

### Flow cytometry analysis

Labelled cells with polyclonal antibodies were acquired and analysed in a MACS Quant Flow Cytometer device (Miltenyi Biotec, Germany) using the following acquisition parameters: flow rate set to "low", uptake volume of 10 µL, FSC set to hyperlogarithmic amplification (370 V), SSC set to hyperlogarithmic amplification (400 V), channel B1 corresponding to the FITC detection set to hyperlogarithmic amplification (370 V) and channel R1 corresponding to the APC detection set to hyperlogarithmic amplification (360 V). At least 10,000 events were acquired in each run.

### Permeabilization of Escherichia coli Nissle 1917 and LMG2092

One mL of culture in exponential phase of growth was centrifuged at 10,000 x g for 5 min. After that, the turbidity of the bacterial suspension was adjusted to an OD600=0.2 (around 1E10⁸ CFUs/mL) using FC buffer. Both *E. coli* were fixed in 4% cold and freshly prepared paraformaldehyde in Phosphate Buffered Saline (PBS). Samples were then incubated at 4° C for 10 min. Bacteria were washed with FC buffer in order to remove extra-fixative reagents. For permeabilization, Tween 20 at concentration of 2% was added to each tube and incubated for 10 min at room temperature. The samples were washed with FC buffer and then, bacteria were stained with antibodies.

### Detection of Escherichia coli Nissle 1917 and LMG2092 by flow cytometry

The binding capability of the four anti-peptides antibodies was tested in a *pks*+ (*E. coli* Nissle 1917) and a *pks*- (*E. coli* LMG2092) strains. Twenty-five µL of the bacterial suspension were mixed with 25 µL of the FITC-conjugated antibody at a final concentration of 20 µg/mL. The samples were incubated for 15 min at RT and then were washed with FC buffer at 13,000 rpm for 5 minutes. Finally, bacteria were resuspended in 150 µL of FC buffer and samples were acquired using a MACS Quant Flow Cytometry (Miltenyi Biotec, Germany). Bacteria were labelled and analysed at different optical density (i.e. OD600 = 0.3, 0.6, 1 and 1.5) and with different medium (i.e. Luria Bertani, Nutrient Broth and Nutrient Broth supplemented with 2% of glucose).

### Detection of pks+ strains in a gut microbiota

The detection of *pks*+ strains was investigated over 5 samples of gut microbiotas from healthy donors and 5 samples from Lynch syndrome patients. Microbiotas were labelled with the antibody of the invention conjugated to APC and incubated for 15 minutes at room temperature. Then, they were washed with FC buffer at 13,000 rpm for 5 minutes and the supernatant was removed. Finally, microbiotas were resuspended in 150 µL of FC buffer and data were acquired and analysed using a MACS Quant Flow cytometer as indicated previously.

### Enrichment and depletion of pks+ strains from a gut microbiota

Microbiotas were labelled with the polyclonal anti-peptide 2 serum IgG fraction conjugated to APC and incubated for 15 minutes at room temperature. Then, they were washed with FC buffer at 13,000 rpm for 5 minutes and the supernatant was removed. For positive selection of *E. coli pks*+*,* microbiotas were resuspended in 100 µL of resuspension buffer (PBS supplemented with 2 mM EDTA and 3% (v/v) of decomplemented bovine foetal serum). To this mix, 10 µL of magnetic anti-APC particles were added (BD Biosciences, San Jose, USA). The mixture was incubated for 15 minutes at 4 °C. Then, microbiotas were washed at 13000 rpm for 5 minutes with 1X BD IMag buffer (BD Biosciences, San Jose, USA) and the column was conditioned with 1X BD IMag buffer (BD Biosciences, San Jose, USA). Positive fraction was retained in the magnetic column after three washes with 1X BD IMag buffer (BD Biosciences). Finally, the positive fraction was eluted through the 1X BD IMag buffer. Both positive and negative fractions were further analysed by flow cytometry using a MACS Quant Flow cytometer as indicated previously.

### Immunofluorescence microscopy

The binding ability of the antibody anti-peptide according to SEQ ID NO: 1, the antibody anti-peptide according to SEQ ID NO: 2 and the antibody anti-peptide according to SEQ ID NO: 3 contained in each of the polyclonal serums was also determined by confocal scanning laser microscope equipped with a Leica DFC365FX digital camera (DMi8, Leica Microsystems). The bacterial species were grown until exponential phase OD600 of 0.7, then were washed with flow cytometry buffer, fixated with paraformaldehyde 4%, permeabilised with flow cytometry buffer supplemented with 2% of Tween 20 and incubated for 15 min with the polyclonal antibody anti-peptides conjugated with FITC (200 µg/mL). Finally, bacteria were washed once with flow cytometry buffer, resuspended in 10 µL of flow cytometer buffer and were analysed with confocal microscopy using a 100x oil objective. The images were acquired with software LasX (Leica Microsystems). The FITC filter cube (excitation 480/40, emission 527/30) was used.

### Validation of the positive fraction by PCR analysis

Strains retrieved in the positive fraction of the magnetic separation were screened for the presence of *pks* islands by PCR using primers for the *clbB* gene, predicted before as present in all the *pks*+ strains. Two different primers for amplify different regions of the *clbB* gene were selected, i.e. clbB1 and clbB2. Primers used to amplify these regions were clbB1r consisting of SEQ ID NO: 8 ((5'-CCATTTCCCGTTTGAGCACAC-3'), clbB1f consisting of SEQ ID NO: 9 (5'-GATTTGGATACTGGCGATAACCG-3'), clbB2r consisting of SEQ ID NO: 10 (5'-GCGCTCTATGCTCATCAACC-3') and clbB2f consisting of SEQ ID NO: 11 (5'-GCGCATCCTCAAGAGTAAATA-3'). For primer ClbB1, the PCR temperature cycling conditions were as follows: initial denaturation for 10 min at 95°C, followed by 30 standard cycles: denaturation at 95°C for 45 secs, primer annealing for 1 min at 54°C, and primer extension at 72°C for 1 min, and a final extension step at 72°C for 5 min. For primer ClbB2, the PCR temperature cycling conditions were as follows: initial denaturation for 7 min at 95°C, followed by 35 standard cycles: denaturation at 95°C for 30 secs, primer annealing for 30 secs at 55°C, and primer extension at 72°C for 30 secs, and a final extension step at 72°C for 7 min.

### 2. Results

### Distribution of the pks island genes

Not all of the *pks*+ *E. coli* strains presented all the genes related to the reference *pks* island described for strain IHE3034. Table 1 shows the distribution of the *pks* islands in the strains where it was detected. Most of the genes, i.e. *clbP, clbO, clbN, clbM, clbL, clbl, clbH, clbF, clbE, clbD, clbC, clbB,* were detected in all the island distributions, However, the intP4, clbS, clbQ, clbK, clbJ, trpA and trpB genes were detected with a percentage of distribution between 97 and 99%, while for the clbR, clbA and trpC genes their percentage were lower, between 70 and 78%, with clbR being the gene detected in only 70% of the strains.

**Table 1. Distribution of the colibactin island genes along the pks+ strains. Fourteen strains containing errors were discarded.**

| **Gene #** | **Gene name** | **Putative function** | **Detection percentage** |
|---|---|---|---|
| 1 | *intP4* | P4-like integrase | 1,198 of 1,212 (98.84%) |
| 2 | *clbS* | Colibactin resistance protein | 1,186 of 1,212 (97.85%) |
| 3 | *clbQ* | Thioesterase | 1,211 of 1,212 (99.92%) |
| 4 | *cblP* | FmtA-like protein | 1,212 of 1,212 (100.0%) |
| 5 | *clbO* | PKS | 1,212 of 1,212 (100.0%) |
| 6 | *clbN* | NRPS | 1,212 of 1,212 (100.0%) |
| 7 | *clbM* | MatE-like protein | 1,212 of 1,212 (100.0%) |
| 8 | *clbL* | Amidase | 1,212 of 1,212 (100.0%) |
| 9 | *clbK* | PKS/NRPS | 1,094 of 1,212 (90.26%) |
| 10 | *clbJ* | NRPS | 1,202 of 1,212 (99.17%) |
| 11 | *clbl* | PKS | 1,212 of 1,212 (100.0%) |
| 12 | *clbH* | NRPS | 1,212 of 1,212 (100.0%) |
| 13 | *clbG* | Malonyl-CoA transacylase | 1,212 of 1,212 (100.0%) |
| 14 | *clbF* | Acyl-CoA-dehydrogenase | 1,226 of 1,226 (100.0%) |
| 15 | *clbE* | Acyl/D-alanyl carrier protein | 1,212 of 1,212 (100.0%) |
| 16 | *clbD* | 3-hydroxyacyl-CoA-dehydrogenase | 1,212 of 1,212 (100.0%) |
| 17 | *clbC* | PKS | 1,212 of 1,212 (100.0%) |
| 18 | *clbB* | NRPS/PKS | 1,212 of 1,212 (100.0%) |
| 19 | *clbR* | LuxR-like | 856 of 1,212 (70.63%) |
| 20 | *clbA* | Phosphopantetheinyl transferase | 968 of 1,212 (79.87%) |
| 21 | *trpA* | IS *1400* transposase ORFA | 1,193 of 1,212 (98.43%) |
| 22 | *trpB* | IS *1400* transposase ORFB | 1,209 of 1,212 (99.75%) |
| 23 | *trpC* | Transposase fragment | 741 of 1,212 (61.13%) |

### Selection of pks+ unique peptides

Four peptides, the peptides of the invention, were selected based on their exclusive belonging to the *pks*+ strains for antibody generation (Table 2). These 20 amino acid length peptides were predicted on 3 cytoplasmic membrane proteins, i.e. clbH (NRPS), clbC (*PKS*) and clbD (NRPS/*PKS*).

**Table 2: Selected peptides for the antibody generation. All the peptides selected were predicted in the cytoplasmatic membrane.**

| **Antibody** | **Peptide sequence** | ***Pks* gene** | **Presence on *pks*- strains** | **Presence on *pks*+ strains** |
|---|---|---|---|---|
| 1 | LDDHGNPVADGEEGELYLAG (SEQ ID NO: 1) | clbH | 2 of 4986 | 1,225 of 1,226 |
| 2 | WRDGESQQIHYIGRNDFQIK (SEQ ID NO: 2) | clbH | 2 of 4986 | 1,225 of 1,226 |
| 3 | AAGDRIYAVIRGSAVNNDGK (SEQ ID NO: 3) | clbC | 2 of 4986 | 1,224 of 1226 |
| 4 | FNTLVVLENYPVDMTLLSCA (SEQ ID NO: 4) | clbB | 2 of 4986 | 1,225 of 1226 |

### Labelling strains with different antibodies

*Escherichia coli* Nissle 1917 and LMG2092 were growth in LB at 37°C, in aeration, until reached optical density OD₆₀₀ of 0.6. Then, bacteria were labelled with four different polyclonal antibodies, targeting each one of the peptides of the invention according to SEQ ID NO: 1 - 4. The labelling of *pks*+ (Nissle 1917) and *pks*- (LMG2092) strains with the antibodies anti-peptide according to SEQ ID NO: 1, anti-peptide according to SEQ ID NO: 2, anti-peptide according to SEQ ID NO: 3 or anti-peptide according to SEQ ID NO: 4, were evaluated by flow cytometry. Figure 1 represents the specificity of the antibody anti-peptide according to SEQ ID NO: 1, the antibody anti-peptide according to SEQ ID NO: 2, the antibody anti-peptide according to SEQ ID NO: 3 or the antibody anti-peptide according to SEQ ID NO: 4 in the detection of *E. coli* Nissle, with 50% approximately of bacteria labelled after 15 minutes of incubation with the anti-peptide according to SEQ ID NO: 1, anti-peptide according to SEQ ID NO: 2 or anti-peptide according to SEQ ID NO: 3 antibodies. The labelling of bacteria with anti-peptide according to SEQ ID NO:4 antibody was less performant, so for the following experiments it was discarded.

### Labelling strains at different conditions

In order to find the best labelling conditions, *Escherichia coli* Nissle 1917 and LMG2092 were growth in different medium (i.e. LB, nutrient broth and nutrient broth supplemented with 2% of glucose), at different optical density (i.e. 0.3, 0.6, 1 and 1.5) and labelled with antibodies of the invention (Table 3). Figure 2 shows the best labelling conditions, that was obtained in labelling the cells with the FITC labelled antibody of the invention in nutrient broth at OD₆₀₀ of 1.00. The higher emission of *E. coli* Nissle 1917 (Figure 1B) and the lower emission of the *E. coli* LMG2092 (Figure 1A) can be deduced comparing the dispersion plots.

**Table 3. Summary data on the percentage of bacteria labelled with different medium, antibodies and optical density. The data show the mean and the standard deviation.**

| **Medium** | **OD₆₀₀** | **Antibody anti-peptide SEQ ID NO: 1** | **Antibody anti-peptide SEQ ID NO: 2** | **Antibody anti-peptide SEQ ID NO: 3** |
|---|---|---|---|---|
| Luria Bertani | 0.3 | 33.58 ± 2.30 | 44.73 ± 3.03 | 46.55 ± 1.45 |
| | 0.6 | 43.18 ± 2.55 | 48.42 ± 0.90 | 51.60 ± 0.94 |
| | 1 | 44.13 ± 0.79 | 50.42 ± 5.84 | 53.93 ± 2.41 |
| | 1.5 | 37.72 ± 2.18 | 35.64 ± 0.60 | 39.64 ± 2.90 |
| Nutrient broth | 0.3 | 48.56 ± 0.74 | 45.36 ± 3.61 | 42.99 ± 6.78 |
| | 0.6 | 40.74 ± 4.63 | 61.37 ± 5.88 | 43.34 ± 1.78 |
| | 1 | 40.26 ± 1.24 | 63.16 ± 8.82 | 38.80 ± 4.40 |
| | 1.5 | 50.22 ± 7.27 | 43.04 ± 0.98 | 46.20 ± 4.91 |
| Nutrient broth + glucose | 0.3 | 45.79 ± 2.26 | 46.74 ± 3.11 | 36.70 ± 5.55 |
| | 0.6 | 46 ± 0.23 | 47.22 ± 1.31 | 53.45 ± 0.88 |
| | 1 | 49.72 ± 10.16 | 53.3 ± 7.77 | 58.74 ± 6.77 |
| | 1.5 | 49.52 ± 5.23 | 62.19 ± 5.97 | 51.43 ± 5.35 |

### Confocal microscopy of the labelled strains

As the antibody of the invention labelled bound to *Escherichia coli* Nissle 1917 cells in a greater proportion than to LMG2092, some immune-staining pictures were performed. The staining of the *E. coli* Nissle 1917 with the antibody of the invention conjugated with FITC was analysed using the confocal laser scanning microscope (Figure 2C).

### Detection, enrichment and depletion of pks+ strains in gut microbiotas

Ten samples of real gut microbiotas (5 from healthy donors and 5 from Lynch syndrome patients) were analysed to detect, enrich and deplete from *pks*+ strains.

Microbiotas were labelled with the antibody of the invention conjugated with APC and further analysed by flow cytometry for *pks*+ strains detection (Figure 3). From the labelled microbiotas, negative and positive fractions were obtained by magnetic separation. The results show that it is possible to deplete the microbiotas from *pks*+ strains in single step using the antibody of the invention. The positive fractions were also cultivated to discover potential false positives in the cells obtained with the magnetic separations. Table 4 shows the identified isolates by 16S sequencing and the *pks*+ validation by PCR analysis of genes of the colibactin cluster.

**Table 4: Isolated strains from the positive fraction of the gut microbiotas.**

| **Isolated** | **16S** | **Identity percentage** | **PCR validation (ClbB1)** | **PCR validation (ClbB2)** |
|---|---|---|---|---|
| 6 | *Escherichia coli* | 99,37% | Positive | Negative |
| 10 | *Klebsiella pneumoniae* | 98,00% | Negative | Positive |
| 20 | *Escherichia coli* | 98,74% | Negative | Negative |
| 26 | *Escherichia coli* | 98,66% | Negative | Positive |
| 44 | *Escherichia coli* | 100% | Positive | Positive |
| 45 | *Enterococcus faecium* | 99,89% | Negative | Negative |
| 65 | *Escherichia coli* | 93,33% | Negative | Negative |
| 71 | *Citrobacter freundii* | 100% | Negative | Positive |
| 79 | *Escherichia coli* | 99,79% | Negative | Negative |
| 81 | *Enterococcus faecium* | 99,68% | Positive | Positive |
| 91 | *Enterobacter cloacae* | 100% | Negative | Negative |
| 95 | *Citrobacter gillenii* | 99,58% | Positive | Positive |
| 108 | *Escherichia coli* | 99,89% | Negative | Negative |
| 114 | *Escherichia coli* | 100% | Positive | Positive |

## Claims

1. An isolated peptide consisting of the amino acid sequence SEQ ID NO: 2.

2. An isolated polynucleotide encoding for the peptide according to claim 1.

3. An antibody that specifically recognizes the peptide according to claim 1.

4. The antibody according to claim 3 comprising a variable region comprising, preferably consisting of, the SEQ ID NO: 7.

5. The antibody according to any of claims 3 to 4 wherein said antibody is conjugated with a detectable label.

6. Pharmaceutical composition comprising the antibody according to any of claims 3 to 5 and preferably a pharmaceutical acceptable adjuvant, a pharmaceutical acceptable vehicle and/or another active principle.

7. A kit comprising the peptide according to claim 1 and/or the antibody according to any of claims 3 to 5.

8. The use of the antibody according to any of claims 3 to 5 or the kit according to claim 7 for the *in vitro* detection in isolated samples of *pks*+ bacteria, preferably of *E. coli pks+.*

9. The use of the antibody according to any of claims 3 to 5 or the kit according to claim 7 for the isolation from isolated samples of *pks*+ bacteria, preferably of *E. coli pks+.*

10. Method for the *in vitro* detection of *pks*+ bacteria comprising:
a. providing an antibody according to any of claims 3 to 5;
b. adding the antibody to a solution containing a sample of interest in sufficient conditions and with sufficient time of incubation to allow the antibody to bind to the target, creating a reaction mixture; and
c. detecting the presence of the bacterial cells bound to the antibody in the reaction mixture.

11. Method for the isolation from isolated samples of *pks*+ bacteria comprising steps a. and b. of the method according to claim 10 and a step c. comprising separating the cells bound to antibodies from the reaction mixture.

12. The antibody according to any of claims 3 to 5 or the pharmaceutical composition according to claim 6 for use as a medicament.

13. The antibody according to any of claims 3 to 5 or the pharmaceutical composition according to claim 6 for use in the treatment or prevention of colorectal cancer.


SEQUENCE LISTING

<110>  CSIC - Consejo Superior de Investigaciones Científicas
       Fundación Científica de la Asociación Española Contra el Cáncer

<120>  Tools and methods to detect and isolate colibactin producing
       bacteria

<130>  1641.1497

<160>  11

<170>  PatentIn version 3.5

<210>  1
<211>  20
<212>  PRT
<213>  Artificial Sequence

<220>
<223>  Isolated peptide from colibactinH protein

<400>  1

Leu Asp Asp His Gly Asn Pro Val Ala Asp Gly Glu Glu Gly Glu Leu
1               5                   10                  15


Tyr Leu Ala Gly
            20


<210>  2
<211>  20
<212>  PRT
<213>  Artificial Sequence

<220>
<223>  Isolated peptide from colibactinH protein

<400>  2

Trp Arg Asp Gly Glu Ser Gln Gln Ile His Tyr Ile Gly Arg Asn Asp
1               5                   10                  15


Phe Gln Ile Lys
            20


<210>  3
<211>  20
<212>  PRT
<213>  Artificial Sequence

<220>
<223>  Isolated peptide from colibactinC protein

<400>  3

Ala Ala Gly Asp Arg Ile Tyr Ala Val Ile Arg Gly Ser Ala Val Asn
1               5                   10                  15

Asn Asp Gly Lys
            20

<210>  4
<211>  20
<212>  PRT
<213>  Artificial Sequence

<220>
<223>  Isolated peptide from colibactinB protein

<400>  4

Phe Asn Thr Leu Val Val Leu Glu Asn Tyr Pro Val Asp Met Thr Leu
1               5                   10                  15

Leu Ser Cys Ala
            20

<210>  5
<211>  1599
<212>  PRT
<213>  Escherichia coli

<400>  5

Met Glu Gln Gln Gly Ile Met Arg Gln Leu Pro Thr Asp Asp Gln Thr
1               5                   10                  15

Ile Val Asp Tyr Leu Tyr Arg Ile Ala Gly Glu Tyr Gly Glu Lys Ala
            20                  25                  30

Ala Val Leu Met Gly Asp Ala Ala Leu Ser Tyr His Asp Leu Asn Ala
        35                  40                  45

Arg Ser Asn Gln Leu Ala His Tyr Leu Arg Gly Leu Gly Ile Gly Glu
    50                  55                  60

Asp Arg Val Val Ala Ile Arg Leu Pro Arg Gly Met Ala Met Leu Ile
65                  70                  75                  80

Ala Ile Phe Ala Ile Val Lys Ala Gly Gly Ala Tyr Leu Pro Leu Ala
                85                  90                  95

Tyr Asn Ala Pro Pro Arg Ser Arg Ile Glu Asn Ile Leu Ser Asn Ser
            100                 105                 110

Gly Ala Val Cys Leu Ile Gly Thr Asp Asp Gly Asp Arg Trp Pro Ile
        115                 120                 125

Pro Arg Val Glu Ile Asp Ser Ala Ala Val Ser Ala Met Pro Thr Thr

130                 135                 140

Asp Leu Arg Tyr Arg Pro His Ala Arg Gln Leu Ala Tyr Ile Ile Tyr
145                 150                 155                 160

Thr Ser Gly Ser Thr Gly Val Pro Lys Gly Val Ala Thr Glu His Ala
                165                 170                 175

Ala Leu Leu Asn Arg Ile Val Trp Met Gln Asn Ala Tyr Pro Ile Ser
            180                 185                 190

Ser Gln Asp Val Leu Phe Gln Lys Thr Val Tyr Thr Phe Asp Val Ser
        195                 200                 205

Val Trp Glu Met Phe Trp Trp Ala Met Tyr Gly Ala Ser Val Val Leu
    210                 215                 220

Leu Pro Ser Gly Leu Glu Ser Asp Pro Arg Thr Leu Ala Arg Leu Ile
225                 230                 235                 240

Gln Arg His Arg Val Ser Val Val His Phe Val Pro Ser Met Leu Asn
                245                 250                 255

Leu Phe Val Glu Tyr Leu Glu Met Lys Gln Asp Pro Arg Leu Thr Ala
            260                 265                 270

Ser Leu Arg Leu Val Phe Ser Ser Gly Glu Lys Leu Thr Val His Ser
        275                 280                 285

Val Ala Arg Phe Tyr Gln Ser Val Ala Gln Gly Asp Leu Ile Asn Leu
    290                 295                 300

Tyr Gly Pro Thr Glu Ala Ala Ile Asp Val Ser His His Arg Cys Leu
305                 310                 315                 320

Arg Gly Tyr Asp Tyr Asp Asp Ile Pro Ile Gly Gln Ala Ile Asp Gly
                325                 330                 335

Cys Arg Leu Tyr Val Leu Asp Asp His Gly Asn Pro Val Ala Asp Gly
            340                 345                 350

Glu Glu Gly Glu Leu Tyr Leu Ala Gly Ile Gly Leu Ala Arg Gly Tyr
        355                 360                 365

Leu Asn Asn Val Ala Leu Thr Asp Arg Cys Phe Thr Ile His Pro Thr
    370                 375                 380

Leu Arg His Leu Gly Lys Pro Glu Arg Leu Tyr Lys Thr Gly Asp Leu
385                 390                 395                 400

Val Trp Arg Asp Gly Glu Ser Gln Gln Ile His Tyr Ile Gly Arg Asn
                405                 410                 415

Asp Phe Gln Ile Lys Ile Arg Gly Leu Arg Val Glu Leu Gly Glu Ile
            420                 425                 430

Glu Ala His Ala Met Arg Phe Pro Gly Val Gln Gln Ala Val Val Val
        435                 440                 445

Ala Asp Gln Asp Asp Pro Asp Asn Gln Leu Ile Tyr Ala Phe Val Val
    450                 455                 460

Ser Ser Val Pro Leu Asn Leu Ala Ala Leu Met Asp Ala Leu Ser Lys
465                 470                 475                 480

Asn Leu Pro Ala Tyr Met Leu Pro Asn Arg Leu Leu Ala Met Ser Glu
                485                 490                 495

Leu Pro Leu Ser Asp Asn Gly Lys Cys Cys Arg Lys Thr Leu Leu Asp
            500                 505                 510

Leu Ala Arg Ala Tyr Ser Ala Ser Arg Val Asp Leu Arg Glu Thr Pro
        515                 520                 525

Ala Val Arg Tyr Leu Pro Leu Ser Ser Ala Gln Ser Ser Met Trp Phe
    530                 535                 540

Met Gln Gln Leu Ala Pro His Thr Ala Leu Tyr Asn Asn Pro Thr Ala
545                 550                 555                 560

Leu Leu Leu Glu Gly Glu Leu Asp Arg Thr Arg Met Asp Gly Ala Ile
                565                 570                 575

Arg Gln Leu Met Ser Arg His Thr Leu Leu Arg Ala Met Ala Glu Thr
            580                 585                 590

His Asn Gly Gln Pro Val Leu Ala Val Pro Gln Cys Val Ser Ser Gln
        595                 600                 605

Ala Leu Leu Thr Ile Val Pro Leu Pro Ser Val Ser Asp Asp Asn Ala
    610                 615                 620

Leu Gln Ala Met Ile Asn Gln Arg Ala Ala His Pro Met Pro Leu Thr
625                 630                 635                 640

Ser Gly Thr Pro Leu Cys Arg Phe Glu Leu Leu Thr Leu Asp Asp Asp
                645                 650                 655

Arg Ser Val Leu Leu Ile His Leu His His Ile Ile Ser Asp Gly Trp
            660                 665                 670

Ser Lys Gly Val Leu Leu Arg Glu Leu Gln Ala Ala Tyr Asn Gly Glu
        675                 680                 685

Ser Leu Thr Pro Glu Pro Leu Leu Glu Tyr Ala Asp Tyr Met Glu Tyr
    690                 695                 700

Gln Glu Glu Trp Arg Gln Ser Asp Ala Tyr Gln Asp Ala Met Arg Tyr
705                 710                 715                 720

Trp Gln Asn Thr Leu Ala Gly Thr Leu Pro Ile Leu Asp Ile Pro Thr
                725                 730                 735

Asp Gln Pro Arg Gln Lys Val Ala Arg Tyr Gln Gly Ala Phe Val Ala
            740                 745                 750

Phe Ala Leu Ser Ala Asn Thr Cys Glu Arg Val Leu Ala Ala Ala Arg
        755                 760                 765

Ala Gln Arg Val Ser Leu Tyr Asn Tyr Leu Leu Thr Ala Phe Val Leu
    770                 775                 780

Leu Leu His Arg Asn Ala Arg Gln Gln Glu Tyr Ile Val Gly Met Pro
785                 790                 795                 800

Ile Ala Ala Arg Leu Thr Lys Glu Gln Glu His Met Ile Ala Pro Leu
                805                 810                 815

Val Asn Val Leu Pro Leu Arg Leu Pro Leu Asp Glu Ala Ala Ser Phe
            820                 825                 830

Ser Glu Leu Val Gln Thr Ile Arg Gly Ile Leu Phe Ala Ala Phe Arg
        835                 840                 845

His Gln Arg Leu Glu Phe Thr Asp Ile Val Arg Ala Val Asn Val Asp
    850                 855                 860

Arg Ser Ala Gly His Phe Pro Ile Tyr Gln Cys Met Phe Gln Leu Asp
865                 870                 875                 880

Asn Met Pro Leu Ala Ser Pro Thr Leu Asn Gly Val Asn Val Thr Pro
                885                 890                 895

Leu Leu Leu Asp Thr Ser Ala Ser Gln Val Asp Ile Ser Leu Ser Met
            900                 905                 910

Gln His Ile Asp Gly Arg Ile Thr Gly Thr Phe Glu Tyr Asp Ala Gly
        915                 920                 925

Leu Tyr Ser Ala Asp Arg Ile Gln His Leu Val Ala Gln Trp Arg Glu
    930                 935                 940

Leu Leu Asp Glu Ala Ser Ser Gln Pro Thr Gln Leu Val Arg Asp Leu
945                 950                 955                 960

Ile Arg Phe Thr Pro Arg Glu His Ala Trp Leu Ala Arg His Asn Ala
                965                 970                 975

Thr Glu Val Ala Leu Pro Pro Val Asp Asn Leu Leu Ala Leu Val Leu
            980                 985                 990

Pro His Cys Gln Gln Arg Pro Thr Gln Val Ala Leu Arg His Ala Asp
        995                 1000                1005

Asp Ala Met Thr Tyr Gly Glu Leu Gln Gln Ala Thr Met Gln Met
    1010                1015                1020

Cys Thr Trp Leu Arg Ala Gln Gly Val Lys Arg Gly Glu Ser Val
    1025                1030                1035

Ala Leu Gln Leu Pro Phe Cys Phe Glu Leu Ile Ile Ala Gln Leu
    1040                1045                1050

Ala Ile Leu Ser Leu Gly Ala Ser Tyr Val Pro Leu Asp Gly Asn
    1055                1060                1065

Ala Pro Ala Ala Arg Asn Ala Leu Ile Leu Ala Gln Ala Thr Pro
    1070                1075                1080

Cys Met Leu Leu Val Ala Gln Pro Leu Glu Ser Pro His Gly Leu
    1085                1090                1095

Thr Ile Pro Trp Val Leu Val Pro Asp Trp Arg Ser Leu Leu Thr
    1100                1105                1110

Glu Ile Pro Asn Leu Pro Val Ser Val Ala Pro Asp Ala Leu Asp
    1115                1120                1125

Cys Asp Ala Val Val Ile Phe Thr Ser Gly Thr Thr Gly Gln Pro

1130                1135                1140

Lys Gly Val Arg Leu Ser Gln Arg Asn Leu Val Asn Leu Thr Ala
    1145                1150                1155

Ser Phe Ile Ser Ser Tyr Gln Val Thr His Gln Asp Val Leu Leu
    1160                1165                1170

Pro Ile Thr Ser Val Ala Ser Ala Ser Phe Val Gly Glu Val Leu
    1175                1180                1185

Pro Leu Leu Ala Ala Gly Gly Thr Leu Val Leu Ala Gln Lys Ala
    1190                1195                1200

Gln Ser Leu Asp Ser Asp Ala Leu Ile Ala Leu Leu Ala Ser Gln
    1205                1210                1215

Arg Val Thr Ile Leu Ser Thr Thr Pro Ser Leu Ser Ala Ser Leu
    1220                1225                1230

Ser Val Leu Ala Gln Ser Met Gly Ser Leu Arg Leu Phe Leu Cys
    1235                1240                1245

Gly Gly Glu Ala Leu Glu Tyr Glu Gln Ile Ala Pro Leu Leu Pro
    1250                1255                1260

His Met Ala Val Val Asn Gly Tyr Gly Leu Thr Glu Ser Gly Ile
    1265                1270                1275

Cys Ser Thr Tyr Phe Pro Val Ala Lys Arg Arg Glu Gln Glu Thr
    1280                1285                1290

Gly Ala Leu Pro Ile Gly Arg Pro Ile Gln Asn Thr Gln Ala Tyr
    1295                1300                1305

Val Val Asp Ala Tyr Asn Arg Leu Val Pro Pro Gly Ala Cys Gly
    1310                1315                1320

Glu Leu Cys Phe Ser Gly Leu Gly Ile Ser Pro Gly Tyr Leu Asp
    1325                1330                1335

Ala Arg Gln Asp Pro Glu Arg Phe Val Glu Leu Pro Glu Tyr Pro
    1340                1345                1350

Gly Val Arg Val Leu Lys Thr Gly Asp Arg Ala Arg Trp Ala Thr
    1355                1360                1365

Asp Gly Met Leu Phe Tyr Leu Gly Arg Gln Asp Arg Gln Val Gln
    1370                1375                1380

Ile Arg Gly Tyr Arg Val Glu Leu Gly Asp Ile Glu Ser Leu Leu
    1385                1390                1395

Lys Gln His Pro Asp Ile Ala Asp Ala Trp Val Asp Val Arg Arg
    1400                1405                1410

Asn Ala Ala Ala Thr Pro Leu Leu Val Ala Phe Tyr Cys Ser Val
    1415                1420                1425

Asn Gly Val Ala Leu Asp Ala Gln Gln Leu Arg Val Trp Leu Ser
    1430                1435                1440

Leu Arg Leu Pro Leu His Met Leu Pro Leu Leu Tyr Val Pro Leu
    1445                1450                1455

Ser Ala Met Pro Leu Gly Val Asn Gly Lys Ile Asp Pro Gln Cys
    1460                1465                1470

Leu Pro Leu Val Asp Leu Arg Gln Leu Glu Gly Pro Gly Glu Tyr
    1475                1480                1485

Val Pro Pro Ala Thr Glu Leu Glu Gln Arg Leu Ala Glu Ile Trp
    1490                1495                1500

Gln Gln Leu Leu Gly Leu Glu Arg Val Gly Thr Thr Thr Asn Phe
    1505                1510                1515

Phe Asp Leu Gly Gly His Ser Leu Leu Leu Val Gln Met Gln Gln
    1520                1525                1530

Tyr Ile Gly Gln Gln Cys Gly Gln His Val Ala Leu Val Asp Leu
    1535                1540                1545

Leu Arg Phe Thr Thr Ile Lys Arg Leu Ala Glu Phe Leu Leu Ala
    1550                1555                1560

Pro Asp Ala Ala Gln Gly Thr Thr Gly Asp Gln Thr Gln Leu Arg
    1565                1570                1575

Ala Ala Lys Gln Arg Leu Ala Phe Gly His Thr Arg Trp Ala Ala
    1580                1585                1590

Thr Thr Asp Ser His His
    1595

<210>  6
<211>  866
<212>  PRT
<213>  Escherichia coli

<400>  6

Met Glu Tyr Ala Ser Glu Met Asn Gly Met Glu Ile Ala Ile Ile Gly
1               5                   10                  15

Met Ala Val Arg Phe Pro Gln Ser Arg Thr Leu His Glu Phe Trp His
            20                  25                  30

Asn Ile Val Gln Gly Lys Glu Cys Val Thr Phe Phe Ser Glu Glu Glu
        35                  40                  45

Leu Leu Ala Glu Gly Val Glu Gln Ser Thr Leu Asp Asn Pro Ala Tyr
    50                  55                  60

Val Arg Ala Lys Pro Tyr Ile Lys Gly Ile Cys Asp Phe Asp Ala Ala
65                  70                  75                  80

Phe Phe Gly Tyr Ser His Lys Glu Ala Gln Thr Leu Asp Pro Lys Ser
                85                  90                  95

Arg Val Leu His Glu Val Ala Tyr His Ala Leu Glu Asp Ala Gly Tyr
            100                 105                 110

Ala Gln Arg Thr Ser Asp Leu Ile Thr Gly Val Phe Val Gly Ala Ser
        115                 120                 125

Glu Asp Val Asp Trp Leu Arg Arg Ser Leu Ser Gln Ile Gly Gly Asp
    130                 135                 140

Ala Leu Asn Arg Phe Glu Ser Gly Ile Tyr Gly His Lys Asp Leu Leu
145                 150                 155                 160

Ala His Leu Ile Ala Tyr Ser Leu Asn Leu Asn Gly Pro Val Tyr Ser
                165                 170                 175

Leu Tyr Thr Ser Cys Ser Thr Ser Leu Ser Ala Thr His Ile Ala Cys
            180                 185                 190

Arg Ser Leu Leu Phe Gly Glu Cys Asp Leu Ala Leu Ala Gly Gly Ile
        195                 200                 205

Thr Ile Asp Leu Pro Gln Lys Ser Gly Tyr Phe Cys Gln Gln Gly Met
    210                 215                 220

Ile His Ser Thr Asp Gly His Cys Arg Pro Phe Asp Ser Gln Ala Ser
225                 230                 235                 240

Gly Thr Leu Phe Gly Asp Gly Ala Gly Val Val Val Leu Arg Arg Leu
                245                 250                 255

Glu Asp Ala Leu Ala Ala Gly Asp Arg Ile Tyr Ala Val Ile Arg Gly
            260                 265                 270

Ser Ala Val Asn Asn Asp Gly Lys Gln Lys Ile Gly Phe Val Ala Pro
        275                 280                 285

Gly His Glu Gly Gln Lys Ala Val Ile Cys Ala Ala Cys His Leu Ala
    290                 295                 300

Glu Val Ser Pro Glu Ser Ile Gly Tyr Val Glu Thr His Gly Thr Gly
305                 310                 315                 320

Thr Arg Ile Gly Asp Leu Ile Glu Phe Ala Ala Leu Thr Glu Ala Phe
                325                 330                 335

Asp Thr Ser His Arg Gln Tyr Cys Ala Leu Gly Ala Val Lys Ala Asn
            340                 345                 350

Ile Gly His Thr His Ala Ala Ala Gly Val Ala Gly Leu Ile Lys Thr
        355                 360                 365

Ala Leu Val Leu His His Arg Thr Ile Pro Pro Leu Ala Asn Tyr Gln
    370                 375                 380

Met Pro Asn Ser Lys Leu Asp Leu Ala His Ser Pro Phe Tyr Ile Pro
385                 390                 395                 400

Ile Gln Pro Gln Glu Trp Pro Ala Ser Arg Met Pro Pro Arg Ala Gly
                405                 410                 415

Val Ser Ser Phe Gly Ile Gly Gly Thr Asn Val His Met Ile Leu Glu
            420                 425                 430

Gly Leu Asn Pro Ala Val Arg Asp Asp His Asp Gln Val Arg Ala Pro
        435                 440                 445

Val Phe Ile Pro Leu Ser Ala Pro Ser Phe Glu Gln Leu Asp Glu Leu
    450                 455                 460

Thr Gln Gln Leu Thr Pro Leu Leu Ala Thr Leu Asp Ala Ser Thr Leu
465                 470                 475                 480

Ala Tyr Thr Gln Gln Val Ala Arg Pro Val Phe Asp Cys Arg Arg Val
                485                 490                 495

Ile Gln Val Glu Asn Asp Gly Thr Gln Ala Met Leu Ala Ser Leu Asp
            500                 505                 510

Asn Leu Met Pro Asp Ala Pro Trp Gly Leu His Cys Pro Asp Leu Arg
        515                 520                 525

Thr Thr Asn Asp Cys Thr Tyr Ala Gln Trp Leu Ala His Ser Ala His
    530                 535                 540

Tyr Gln Arg Glu Ala Thr Ala Leu Thr Ala Leu Leu Asp Gly Met Asn
545                 550                 555                 560

Ile Pro Pro Ala Tyr Cys His Ala Glu Thr Trp Ala Ala Gln Ala Asn
                565                 570                 575

Ser Ser Leu Leu Ile Arg Gly Cys Gln Thr Ile Ala Ala Leu Lys Thr
            580                 585                 590

Trp Met Asn Leu Leu Pro Thr Leu Thr Leu Leu Ser Gly Ala Gly Thr
        595                 600                 605

Gly Leu Leu Pro Ala Ala Ala Ala Ser Gly Met Ile Ala Thr Gln Asp
    610                 615                 620

Val Leu His Leu Leu Trp Glu Met Glu Gln Lys Ala Leu His Leu Trp
625                 630                 635                 640

Leu Pro Glu Arg His Glu Pro Ile Pro Gly Tyr Val Leu Ala Trp Gln
                645                 650                 655

Gly Asn Pro Ile Thr Asp Ala Gln Arg Asn Asp Arg Gly Phe Trp Ser
            660                 665                 670

Glu Ala Leu Leu Ala Asp Thr Arg Glu Leu Gly Glu Gly Val His Ser
        675                 680                 685

Ile Asn Trp Val Arg Leu Pro Pro Glu Ile Arg Glu Asp Val Asp Val
    690                 695                 700

Leu Arg Tyr Val Ala Gln Leu Trp Cys Ala Gly Ile Asn Val Asp Trp
705                 710                 715                 720

Ala Val Trp Tyr Gly Thr Pro Leu Pro Gln Arg Gly Ser Ala Ser Ala

725                 730                 735

Tyr Pro Phe Ala His Asn His Tyr Pro Leu Pro Gly Arg Val Met Gly
            740                 745                 750

Ser Val Glu Thr Gln Pro Glu Ala Gly Pro Glu Thr His His Pro Tyr
        755                 760                 765

Gln Ala Arg Pro Val Leu Ser Val Pro Phe Val Ala Ala His Ser Arg
    770                 775                 780

Gly Met Gln Tyr Ile Thr Gly Leu Met Glu Leu Leu Leu Glu Ile Ser
785                 790                 795                 800

Pro Val Gly Val Asp Asp Asp Phe Phe Glu Leu Gly Gly His Ser Leu
                805                 810                 815

Leu Val Thr Gln Leu Thr Ser Arg Leu Glu Arg Asp Phe Asn Val His
            820                 825                 830

Ile Asp Leu Leu Thr Leu Met Glu Asn Pro Asn Pro Arg Asn Ile Tyr
        835                 840                 845

Ala His Ile Ala Ala Gln Leu Gly Gly Glu Asp Asn Leu Glu Ile Ala
    850                 855                 860

Cys Gln
865

<210>  7
<211>  20
<212>  PRT
<213>  Artificial sequence

<220>
<223>  Variable region of antibody of invention

<400>  7

Trp Arg Asp Gly Glu Ser Gln Gln Ile His Tyr Ile Gly Arg Asn Asp
1               5                   10                  15

Phe Gln Ile Lys
            20

<210>  8
<211>  21
<212>  DNA
<213>  Artificial Sequence

<220>

<223>  ClbB1 reverse primer 1

<400>  8
ccatttcccg tttgagcaca c                                                21


<210>  9
<211>  23
<212>  DNA
<213>  Artificial Sequence

<220>
<223>  clbB1 forward primer 1

<400>  9
gatttggata ctggcgataa ccg                                              23


<210>  10
<211>  20
<212>  DNA
<213>  Artificial Sequence

<220>
<223>  clbB2 reverse primer 1

<400>  10
gcgctctatg ctcatcaacc                                                  20


<210>  11
<211>  21
<212>  DNA
<213>  Artificial Sequence

<220>
<223>  clbB2 forward primer 1

<400>  11
gcgcatcctc aagagtaaat a                                                21